(19) Europäisches Patentamt
European Patent Office
Office européen des brevets

(11) Veröffentlichungsnummer: **0 252 006**
B1

(12) **EUROPÄISCHE PATENTSCHRIFT**

(45) Veröffentlichungstag der Patentschrift: **28.02.90**

(51) Int. Cl.[4]: **C07C 321/18,** C09K 15/14, C08K 5/37, C10M 135/24

(21) Anmeldenummer: **87810351.4**

(22) Anmeldetag: **22.06.87**

(54) **Neue substituierte Phenole und deren Verwendung als Stabilisatoren.**

(30) Priorität: **26.06.86 CH 2589/86**

(43) Veröffentlichungstag der Anmeldung:
**07.01.88 Patentblatt 88/1**

(45) Bekanntmachung des Hinweises auf die Patenterteilung:
**28.02.90 Patentblatt 90/9**

(84) Benannte Vertragsstaaten:
**DE FR GB IT**

(56) Entgegenhaltungen:
**EP-A- 0 011 091**
**EP-A- 0 145 658**
**EP-A- 0 165 209**
**EP-A- 0 170 624**
**US-A- 3 660 352**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel(CH)**

(72) Erfinder: **Pitteloud, Rita, Dr., av. Général-Guisan 44, CH-1700 Fribourg(CH)**
Erfinder: **Dubs, Paul, Dr., Route des Pralettes 25, CH-1723 Marly(CH)**

**EP 0 252 006 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft neue substituierte 3,5-Bis(merkaptomethyl)-phenole, ein Verfahren zu deren Herstellung sowie deren Verwendung als Stabilisatoren für organisches Material.

Substituierte 3,5-Bis(merkaptomethyl)-phenole sind bekannt. So sind beispielsweise in der DE-A 2 838 273 substituierte Bis(alkylthiomethyl)- bzw. Bis(arylthiomethyl)-phenole als Zwischenprodukte für Pflanzenschutzmittel beschrieben.

Ferner sind aus der US-A 3 660 352 2,4,6-Trialkylphenole, die in 3- oder 3,5-Stellung durch eine höhere Alkyl- oder Alkylbenzylthiomethylgruppe substituiert sind, als Antioxidantien für organische Materialien bekannt. Die genannten Alkyl- oder Alkylbenzylthiomethylgruppen weisen im Alkylteil mindestens 8 C-Atome auf. Spezifisch erwähnt sind nur 2,4,6-Trialkylphenole mit geradkettigen höheren Alkyl- oder Alkylbenzylthiomethylgruppen in 3- oder 3,5-Stellung.

Es besteht jedoch weiterhin ein Bedarf an wirksamen Stabilisatoren für organische Materialien, welche gegen thermischen, oxidativen oder lichtinduzierten Abbau empfindlich sind.

Gegenstand vorliegender Erfindung sind daher Verbindungen der Formel I

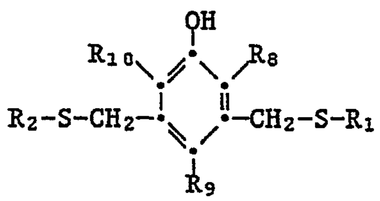 (I),

worin $R_1$ und $R_2$ unabhängig voneinander Wasserstoff, $C_1$-$C_{20}$-Alkyl, $C_3$-$C_{20}$-Alkenyl, $C_5$-$C_{12}$-Cycloalkyl, Phenyl, 1-Naphthyl, 2-Naphthyl, $C_1$-$C_4$_Alkyl-Phenyl, Phenyl-$C_1$-$C_4$-Alkyl oder eine Gruppe der Formeln Ia, Ib oder Ic

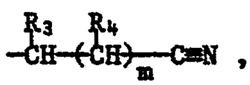 , 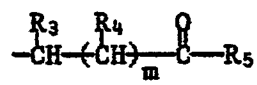 oder 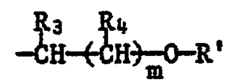

(Ia) (Ib) (Ic)

bedeuten, wobei für den Fall, dass $R_1$ und $R_2$ gleichzeitig für Alkyl stehen, mindestens einer der beiden Substituenten ein mindestens ein tertiäres C-Atom enthaltendes verzweigtes Alkyl bedeutet, oder $R_1$ und $R_2$ eine Gruppe der Formel Id

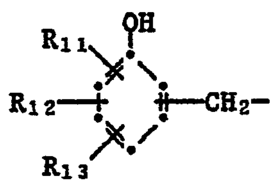 (Id)

die gleich oder verschieden sein können, bedeuten,

m Null oder 1 ist,

$R_3$ und $R_4$ unabhängig voneinander für Wasserstoff oder Methyl stehen,

$R_5$ einen Rest -$OR_6$ oder -$NR_6R_7$ bedeutet,

R' für Wasserstoff oder -CO-$R_6$ steht,

$R_6$ und $R_7$ unabhängig voneinander für Wasserstoff, ein gegebenenfalls durch -O-, -S-, -NH-, -N($CH_3$)- oder -N($CH_2CH_3$)- unterbrochenes $C_1$-$C_{20}$-Alkyl, $C_2$-$C_{20}$-Alkenyl, $C_5$-$C_{12}$-Cycloalkyl, Phenyl, 1-Naphthyl, 2-Naphthyl, Phenyl-$C_1$-$C_4$-Alkyl oder $C_1$-$C_4$-Alkyl-Phenyl stehen, $R_8$, $R_9$ und $R_{10}$ unabhängig voneinander $C_1$-$C_{10}$-Alkyl, $C_2$-$C_{10}$-Alkenyl, $C_5$-$C_{12}$-Cycloalkyl, Phenyl, 1-Naphthyl, 2-Naphthyl, $C_1$-$C_4$-Alkyl-Phenyl oder Phenyl-$C_1$-$C_4$-Alkyl bedeuten,

$R_{11}$ $C_1$-$C_{10}$-Alkyl, $C_2$-$C_{10}$-Alkenyl, $C_5$-$C_{12}$-Cycloalkyl, $C_1$-$C_4$-Alkyl-Phenyl oder Phenyl-$C_1$-$C_4$-Alkyl ist, und

$R_{12}$ und $R_{13}$ unabhängig voneinander für Wasserstoff oder eine Bedeutung von $R_{11}$ stehen.

In Formel I sind $R_1$ und $R_2$ vorzugsweise von Wasserstoff verschieden. $R_1$, $R_2$, $R_6$ und $R_7$ als $C_1$-$C_{20}$-Alkyl und $R_8$, $R_9$, $R_{10}$, $R_{11}$, $R_{12}$ und $R_{13}$ als $C_1$-$C_{10}$-Alkyl bedeuten beispielsweise Methyl, Ethyl, n-Propyl, Isopropyl, n-Butyl, sek.-Butyl, t-Butyl, n-Pentyl, Isopentyl, n-Hexyl, 1,1-Dimethylbutyl, 1,1,3,3-Tetramethylbutyl, 1,1,3,3,5,5-Hexamethylhexyl, n-Heptyl, n-Octyl, 2-Ethylhexyl oder n-Decyl. Bevorzugt sind $R_8$, $R_9$, $R_{10}$, $R_{11}$, $R_{12}$ und $R_{13}$ als Alkyl $C_1$-$C_8$-Alkyl, insbesondere $C_1$-$C_4$-Alkyl.

$R_1$, $R_2$, $R_6$ und $R_7$ können darüber hinaus z.B. 1-Methylundecyl, n-Dodecyl, Tetradecyl, Octadecyl oder Eicosyl bedeuten. Bevorzugt sind $R_1$, $R_2$, $R_6$ und $R_7$ als Alkyl $C_4$-$C_{20}$-Alkyl.

$R_6$ und $R_7$ als gegebenenfalls durch -O-, -S-, -N($CH_3$)- oder -N($CH_2CH_3$)- unterbrochenes $C_1$-$C_{20}$-Alkyl können beispielsweise 2-Methyloxyethylen, 2-Ethyloxypropylen, 3-Ethyloxypropylen, 3,6-Dioxaheptyl, 3,6,9-Trioxaundecyl, 4-Thiahexyl, 11-(Methylaza)-dodecyl, 3-(Ethylaza)-octadecyl bedeuten. Bevorzugt sind $R_6$ und $R_7$ durch -O- unterbrochenes $C_2$-$C_8$-Alkyl.

$R_1$, $R_2$, $R_6$ und $R_7$ als $C_3$-$C_{20}$-Alkenyl bzw. $R_8$, $R_9$, $R_{10}$, $R_{11}$, $R_{12}$ und $R_{13}$ als $C_2$-$C_{10}$-Alkenyl bedeuten beispielsweise Vinyl, Allyl, Methallyl, Penten-1-yl, 3,3-Dimethylpenten-1-yl, Octen-1-yl oder Decen-1-yl. Bevorzugt is Allyl. $R_1$, $R_2$, $R_6$ und $R_7$ können darüber hinaus beispielsweise Dodecen-1-yl oder Octadecen-1-yl sein.

Als $C_5$-$C_{12}$-Cycloalkyl in den Resten $R_1$, $R_2$, $R_6$, $R_7$, $R_8$, $R_9$, $R_{10}$, $R_{11}$, $R_{12}$ und $R_{13}$ sind z.B. Cycloalkylreste mit 5-8, insbesondere 5 oder 6, Ring-C-Atomen zu verstehen, die gegebenenfalls durch $C_1$-$C_4$-Alkyl substituiert sein können. Beispiele für derartige Reste sind Cyclopentyl, Methylcyclopentyl, Cyclohexyl, Methylcyclohexyl, Ethylcyclohexyl, Cycloheptyl und Cyclooctyl. Besonders zu erwähnen sind die unsubstituierten Cycloalkylreste. Bevorzugt bedeutet Cycloalkyl jedoch gegebenenfalls durch $C_1$-$C_4$-Alkyl substituiertes Cyclohexyl, insbesondere Cyclohexyl oder Methylcyclohexyl, vor allem Cyclohexyl.

Von den Bedeutungen Phenyl, 1-Naphthyl und 2-Naphthyl für $R_1$, $R_2$, $R_6$, $R_7$, $R_8$, $R_9$, $R_{10}$ ist Phenyl bevorzugt.

$R_1$, $R_2$, $R_6$, $R_7$, $R_8$, $R_9$, $R_{10}$, $R_{11}$, $R_{12}$ und $R_{13}$ können als $C_1$-$C_4$-Alkyl-Phenyl beispielsweise Tolyl, Xylyl oder 4-t-Butylphenyl bedeuten. Phenyl kann dabei vorzugsweise durch 1 bis 3 Alkylreste substituiert sein.

$R_1$, $R_2$, $R_6$, $R_7$, $R_8$, $R_9$, $R_{10}$, $R_{11}$, $R_{12}$ und $R_{13}$ können als Phenyl-$C_1$-$C_4$-Alkyl beispielsweise Benzyl, α-Methylbenzyl, α,α-Dimethylbenzyl oder α-Methyl-α-ethylbenzyl bedeuten. Bevorzugt ist Benzyl.

Zu erwähnen sind besonders Verbindungen der Formel I

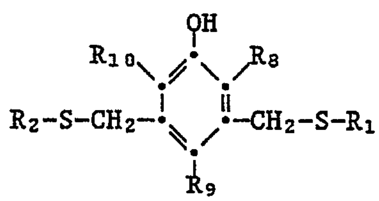

(I),

worin $R_1$ und $R_2$ unabhängig voneinander $C_1$-$C_{20}$-Alkyl, $C_3$-$C_{20}$-Alkenyl, $C_5$-$C_{12}$-Cycloalkyl, Phenyl, 1-Naphthyl, 2-Naphthyl, $C_1$-$C_4$-Alkyl-Phenyl, Phenly-$C_1$-$C_4$-Alkyl oder eine Gruppe der Formeln Ia, Ib oder Ic

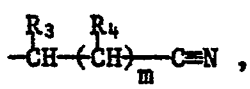

(Ia), 

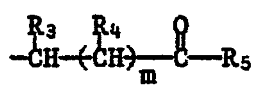

(Ib) oder

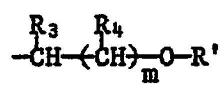

(Ic)

bedeuten, wobei für den Fall, dass $R_1$ und $R_2$ gleichzeitig für Alkyl stehen, mindestens einer der beiden Substituenten ein mindestens ein tertiäres C-Atom enthaltendes verzweigtes Alkyl bedeutet, m Null oder 1 ist, und

R', $R_3$, $R_4$, $R_5$, $R_6$, $R_7$, $R_8$, $R_9$, $R_{10}$ die zuvor genannte Bedeutung haben.

Bevorzugt sind Verbindungen der Formel I, worin $R_1$ und $R_2$ unabhängig voneinander eine Gruppe der Formel Id bedeuten, und insbesondere diejenigen, worin $R_{11}$ und $R_{12}$ in der Formel Id für $C_1$-$C_{10}$-Alkyl stehen.

Ebenfalls zu erwähnen sind Verbindungen Formel I, worin $R_1$ $C_3$-$C_{20}$-Alkenyl, $C_5$-$C_{12}$-Cycloalkyl, Phenyl, 1-Napthyl, 2-Naphthyl, $C_1$-$C_4$-Alkyl-Phenyl, Phenyl-$C_1$-$C_4$-Alkyl oder eine Gruppe der Formeln Ia, Ib oder Ic bedeutet und $R_2$ $C_1$-$C_{20}$-Alkyl ist oder unabhängig von $R_1$ eine für $R_1$ genannte Bedeutung hat.

Ferner sind Verbindungen der Formel I hervorzuheben, worin $R_1$ und $R_2$ die gleiche Bedeutung haben und für Wasserstoff, $C_6$-$C_{20}$-Alkyl mit mindestens einem tertiären C-Atom, eine Gruppe der Formel Ib oder eine Gruppe der Formel Id stehen, insbesondere solche, in denen in Formel Ib $R_3$ und $R_4$ Wasserstoff sind und $R_5$ für einen Rest $OR_6$ steht, worin $R_6$ $C_6$-$C_{18}$-Alkyl bedeutet und in Formel Id $R_{11}$ $C_1$-$C_6$-Alkyl und $R_{12}$ und $R_{13}$ unabhängig voneinander Wasserstoff oder $C_1$-$C_6$-Alkyl bedeuten.

Von Bedeutung sind Verbindungen der Formel I, worin $R_1$ und $R_2$ die gleiche Bedeutung haben und für $C_6$-$C_{20}$-Alkyl mit mindestens einem tertiären C-Atom stehen.

Ebenfalls von Bedeutung sind Verbindungen der Formel I, worin $R_1$ und $R_2$ unabhängig voneinander eine Gruppe der Formel Ic darstellen.

Bevorzugt sind Verbindungen der Formel I, worin $R_1$ $C_5$-$C_{12}$-Cycloalkyl, Benzyl, Phenyl oder eine Gruppe der Formeln Ia oder Ib bedeutet und $R_2$ $C_4$-$C_{20}$-Alkyl oder unabhängig von $R_1$ eine für $R_1$ ge-

nannte Bedeutung hat, und insbesondere solche, worin $R_1$ und $R_2$ unabhängig voneinander eine Gruppe der Formel Ib bedeuten.

Besonders bevorzugt sind Verbindungen der Formel I, worin $R_1$ und $R_2$ unabhängig voneinander eine Gruppe

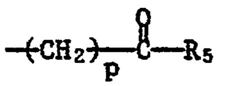 oder 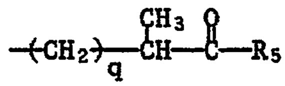

bedeuten, worin p für eine Zahl 1 oder 2 steht, q Null oder 1 bedeutet und $R_5$ die zuvor genannte Bedeutung hat insbesondere solche, worin $R_5$ $-OR_6$ oder $NR_6R_7$ ist, $R_6$ ein gegebenenfalls durch -O- unterbrochenes $C_1$-$C_{18}$-Alkyl, $C_5$-$C_{12}$-Cycloalkyl, Phenyl, 1-Naphthyl, 2-Naphthyl, Phenyl-$C_1$-$C_4$-Alkyl oder $C_1$-$C_4$-Alkyl-Phenyl bedeutet, $R_7$ unabhängig von $R_6$ eine für $R_6$ angegebene Bedeutung hat, und $R_8$, $R_9$, $R_{10}$ unabhängig voneinander $C_1$-$C_8$-Alkyl, $C_5$-$C_{12}$-Cycloalkyl, Phenyl oder Benzyl sind. Bevorzugt bedeuten $R_8$, $R_9$ und $R_{10}$ $C_1$-$C_8$-Alkyl, insbesondere $C_1$-$C_4$-Alkyl, wobei sie vorzugsweise die gleiche Bedeutung haben.

In besonders bevorzugten Verbindungen der Formel I sind $R_1$ und $R_2$ gleich.

Es sind weiter Verbindungen der Formel I zu erwähnen, worin $R_1$ und $R_2$ gleich sind und jeweils eine Gruppe der Formel Id bedeuten. Die OH-Gruppe steht vorzugsweise in meta-Stellung zur $-CH_2$-S-Verknüpfung.

Von besonderem Interesse sind Verbindungen der Formel I, worin $R_1$ und $R_2$ $-CH_2-COO-CH_2-CH(CH_2CH_3)-(CH_2)_3-CH_3$ bedeuten, und $R_8$, $R_9$ und $R_{10}$ für Methyl stehen.

Ebenfalls von besonderem Interesse sind Verbindungen der Formel I, worin $R_1$ und $R_2$ Wasserstoff bedeuten.

Als Beipiele für Vertreter der erfindungsgemässen Verbindungen der Formel I seien genannt:

3,5-Bis(merkaptomethyl)-2,4,6-trimethyl-phenol,
3,5-Bis(2'-hydroxyethylthiomethyl)-2,4,6-trimethyl-phenol,
3,5-Bis(3'-methylamido-propylthiomethyl)-2,4,6-trimethyl-phenol,
3,5-Bis(3'-dimethylamido-propylthiomethyl)-2,4,6-trimethyl-phenol,
3,5-Bis(1'-cyanoethylidenthiomethyl)-2,4,6-trimethyl-phenol,
3,5-Bis(4'-n-decanoyloxybutyl-thiomethyl)-2,4,6-trimethyl-phenol,
3-(t-Octylthiomethyl)-5-(benzylthiomethyl)-2,4,6-trimethyl-phenol,[1]
3-(t-Dodecylthiomethyl)-5-(2'-acetyloxyethyl-thiomethyl)-2,4,6-trimethyl-phenol,[2]
3,5-Bis(benzylthiomethyl)-2,4,6-trimethyl-phenol,
3,5-Bis(phenylthiomethyl)-2,6-dimethyl-4-t-butyl-phenol,
3,5-Bis(napht-1-yl-thiomethyl)-2,4,6-trimethyl-phenol,
3,5-Bis(2'-ethoxycarbonylmethyl-thiomethyl)-2,4,6-trimethyl-phenol,
3,5-Bis(n-octadecyloxycarbonylmethyl-thiomethyl)-2,4,6-trimethyl-phenol,
3,5-Bis[(3'-methyl-4'-hydroxy-5'-t-butyl)benzyl-thiomethyl]-2,4,6-trimethylphenol.

[1] t-Octyl ist 1,1,3,3-Tetramethylbutyl
[2] t-Dodecyl ist ein Gemisch aus 1,1,3,3,5,5-Hexamethylhexyl und 1,1,4,6,6-Pentamethylhept-4-yl

Bei der an sich bekannten Herstellung der erfindungsgemässen Verbindungen der Formel I geht man beispielsweise so vor, dass man eine Verbindung der Formel II

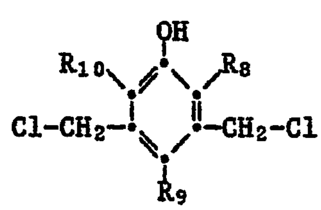 (II)

mit einem Merkaptan $R_1$-SH oder mit einem Gemisch von $R_1$-SH/$R_2$-SH in Gegenwart einer Base umsetzt, wobei $R_1$, $R_2$, $R_8$, $R_9$ und $R_{10}$ die zuvor genannte Bedeutung haben.

Im Falle von Mischungen $R_1$-SH/$R_2$-SH ist das Mischungsverhältnis bevorzugt 1:1.

Als Basen eignen sich organische und anorganische Basen. Geeignete organische Basen sind beispielsweise tertiäre Amine, wie zum Beispiel Triethylamin, Pyridin oder N,N-Dimethylanilin, oder Alkoholate, wie zum Beispiel Natriumethylat. Geeignete anorganische Basen, in fester oder flüssiger Form, sind beispielsweise Carbonate, wie zum Beispiel Natriumhydrogencarbonat, Kaliumcarbonat oder Alkali- und Erdalkalihydroxide, wie zum Beispiel Natriumhydroxid oder Kaliumhydroxid.

Die Umsetzung kann vorteilhafterweise im Temperaturbereich von -20°C bis 160°C durchgeführt werden. Bevorzugt ist der Temperaturbereich von 0°C bis 80°C.

Die Umsetzung kann vorteilhafterweise während 0,5 bis 3 Stunden unter $N_2$-Atmosphäre im angegebenen Temperaturbereich erfolgen, wobei die Temperatur so gewählt wird, dass das Reaktionsgemisch am Rückfluss erhitzt wird.

Die erfindungsgemässen Verbindungen der Formel I können zum Beispiel auch dadurch gewonnen werden, dass man eine Verbindung der Formel

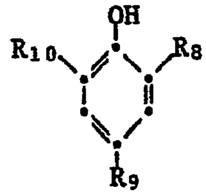

mit einer Verbindung $R_1$-X oder mit einem Gemisch von $R_1$-S-$CH_2$-X/$R_2$-S-$CH_2$-X in Gegenwart eines Katalysators umsetzt, wobei $R_1$, $R_2$, $R_8$, $R_9$ und $R_{10}$ die zuvor genannte Bedeutung haben. X steht für Halogen, Tosyl, Mesyl, OH, Alkoxy oder Acyloxy wie z.B. Acetoxy.

Als Katalysatoren eignen sich Brönstedt- oder Lewis-Säuren, wie zum Beispiel $ZnCl_2$ oder $SnCl_4$.

Die erfindungsgemässen Verbindungen der Formel I, worin $R_1$=$R_2$=H bedeuten,

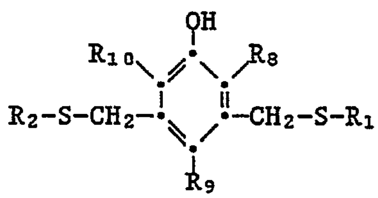

(I)

können beispielsweise dadurch hergestellt werden, dass man eine Verbindung der Formel II mit Thioharnstoff umsetzt. Die Symbole $R_8$, $R_9$ und $R_{10}$ haben die zuvor genannte Bedeutung.

Die Umsetzung kann vorteilhafterweise in Gegenwart eines geeigneten Lösungsmittels, wie z.B. eines Aethers, während 0,5 bis 6 Stunden unter $N_2$-Atmosphäre in einem Temperaturbereich von 80 bis 250°C erfolgen, wobei die Temperatur so gewählt wird, dass das Reaktionsgemisch am Rückfluss siedet.

Die erfindungsgemässen Verbindungen der Formel I, worin $R_1$ und $R_2$ unabhängig voneinander eine Gruppe der Formel Id

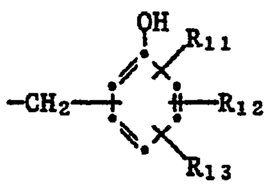

(Id)

bedeuten, und $R_{11}$, $R_{12}$ und $R_{13}$ die zuvor genannte Bedeutung haben, können beispielsweise dadurch hergestellt werden, dass man eine erfindungsgemässe Verbindung der Formel

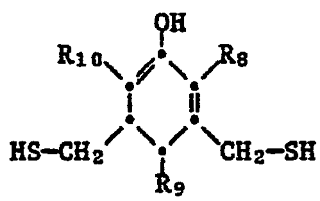

mit einer phenolischen Mannichbase der Formel

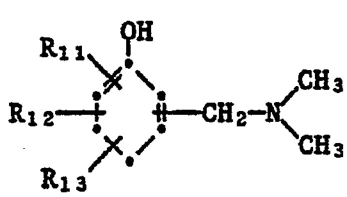

,

oder deren Mischungen, umsetzt. Die Symbole $R_8$, $R_9$ und $R_{10}$ haben die zuvor genannte Bedeutung.

Die Umsetzung kann vorteilhafterweise im Temperaturbereich von 80°C bis 250°C, bevorzugt von 100°C bis 180°C, während 0,5 bis 5 Stunden unter $N_2$-Atmosphäre durchgeführt werden.

Die erfindungsgemässen Verbindung der Formel I, worin $R_1$ und $R_2$ unabhängig voneinander eine Gruppe der Formel Id bedeuten, können beispielsweise auch so gewonnen werden, dass man eine erfindungsgemässe Verbindung der Formel

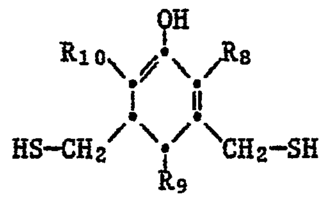

mit einer Verbindung der Formel

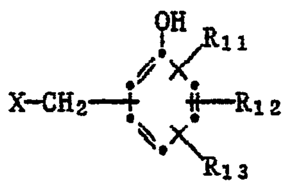

in Gegenwart einer Base umsetzt.

X steht für eine Abgangsgruppe, wie z.B. Halogen, Acetat, Tosylat oder Mesylat, und die Symbole $R_8$, $R_9$, $R_{10}$, $R_{11}$, $R_{12}$ und $R_{13}$ haben die zuvor genannte Bedeutung.

Die geeigneten Basen und vorteilhaften Bedingungen für die Umsetzung sind zuvor beschrieben worden.

Die Umsetzungen können in Gegenwart eines Lösungsmittels erfolgen. Geeignete Lösungsmittel sind aliphatische, cycloaliphatische oder aromatische Kohlenwasserstoffe, wie zum Beispiel Hexan, Cyclohexan oder Toluol, halogenierte Kohlenwasserstoffe, wie zum Beispiel Methylenchlorid oder Chloroform, Alkohole, Aether sowie polare aprotische Lösungsmittel, wie zum Beispiel Dimethylformamid. Es können auch Gemische der genannten Lösungsmittel eingesetzt werden.

Die erfindungsgemässen Verbindungen der Formel I werden zweckmässig mit einer geeingneten Methode, wie zum Beispiel Filtration und/oder Waschen mit wässriger Salzssäure, von allenfalls anfallenden Hydrochloriden oder anderen festen Rückständen abgetrennt und durch Abdestillieren oder Eindampfen des Lösungsmittels (gegebenenfalls bei reduziertem Druck) isoliert. Sie können durch geeeignete Reinigungsmethoden, z.B. Umkristallisation oder Säulenchromatographie weiter gereinigt werden.

Wird die Umsetzung mit Mischungen von Merkaptanen $R_1$-SH/$R_2$-SH durchgeführt, so werden in der Regel statistische Mischungen von symmetrisch und unsymmetrisch substituierten Endprodukten der Formel I erhalten. Diese können nach üblichen Trennungsmethoden (z.B. nach den im vorstehenden Absatz beschriebenen) in die Einzelkomponenten aufgetrennt werden. Sie können aber auch als Mischungen direkt als Stabilisatoren eingesetzt werden.

Die Ausgangsprodukte der Formel II, die phenolischen Mannichbasen sowie die Merkaptane $R_1$-SH und $R_2$-SH sind bekannte Verbindungen und können nach bekannten Verfahren hergestellt werden. Sie sind zum Teil auch im Handel erhältlich.

Die erfindungsgemässen Verbindungen sind wirksame Stabilisatoren für organische Materialien, welche gegen thermischen, oxidativen oder strahlungsinduzierten Abbau empfindlich sind.

Ein weiterer Gegenstand der Erfindung ist daher die Verwendung der erfindungsgemässen Verbindungen der Formel I als Stabilisatoren gegen thermischen, oxidativen oder strahlungsinduzierten Abbau in einem organischen Material.

Bevorzugt ist die Verwendung der erfindungsgemässen Verbindungen der Formel I als Antioxidantien in synthetischen Polymeren, Z.B. thermoplastischen Kunststoffen oder Elastomeren, oder die Verwendung in Schmierstoffen, z.B. in Mineralölen oder synthetischen Oelen. Besonders bevorzugt ist die Verwendung in Polyolefinen, z.B. in Polypropylen.

Beispiele für organische Materialien, welche mit in den erfindungsgemässen Verbindungen der Formel I vorteilhaft stabilisiert werden können, sind:

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B.

Ethylen-Propylen-Copolymere, Propylen-Buten-1-Copolymere, Propylen-Isobutylen-Copolymere, Ethylen-Buten-1-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5.Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril-Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenohlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten.

13. Polyphenyloxide und -sulfide und deren Mischungen mit Styrolpolymeren.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylenisophthalamid, sowie deren Block-Copolymere mit Polyethern, wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesterharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren, wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Oele, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die in Frage kommenden Scmierstoffe sind dem Fachmann geläufig und z.B. im "Schmiermittel Taschenbuch" (Hüthig Verlag, Heidelberg, 1974) beschrieben.

Die erfindungsgemassen Stabilisatoren werden den Polymeren bzw. den Schmiermitteln zweckmässig in einer Konzentration von 0,01-10 Gew.-%, berechnet auf das zu stabilisierende Material, zugesetzt. Vorzugsweise werden 0,05 bis 5,0 Gew.-%, besonders bevorzugt 0,1 bis 2,0 Gew.-% der Verbindungen der Formel I, berechnet auf das zu stabilisierende Material, in dieses eingearbeitet.

Die Einarbeitung kann beispielsweise durch Einmischen der erfindungsgemässen Stabilisatoren und gegebenefalls weiterer Additive nach den in der Technik üblichen Methoden, vor oder während der Formgebung, oder auch durch Aufbringen der gelösten oder dispergierten Verbindungen auf das Polymere, gegebenenfalls unter nachträglichem Verdunsten des Lösungsmittels, erfolgen. Die erfindungsgemässen Stabilisatoren können auch in Form eines Masterbatches, der den Stabilisator beispielsweise in einer Konzentration von 2,5 bis 25 Gew.-% enthält, den zu stabilisierenden Kunststoffen zugesetzt werden.

Die Verbindungen der Formel I können auch vor oder während der Polymerisation oder der Vernetzungsreaktion zugefügt werden. Man erhält so direkt stabilisierte Polymere.

Die so stabilisierten Materialien können in verschiedenster Form angewendet werden, z.B. als Folien, Fasern, Bändchen, Formmassen, Profile oder als Bindemittel für Lacke, Klebstoffe oder Kitte.

Ein weiterer Gegenstand der vorliegenden Erfindung betrifft Zusammensetzungen enthaltend ein gegen thermischen, oxidativen oder strahlungsinduzierten Abbau enpfindliches organisches Material und als Stabilisator mindestens eine Verbindungen der Formel I. Beispiele für derartige organische Materialien sind vorstehend aufgezählt.

Bevorzugt werden Zusammensetzungen, worin das organische Material ein synthetisches Polymeres ist.

Die Polymere können auch in Form von Latices vorliegen und können als solche stabilisiert sein.

Bevorzugt ist das Polymer ein thermoplastischer Kunststoff oder ein Elastomer, vor allem ein Polyolefin, z.B. Polypropylen.

Ebenfalls bevorzugt werden Zusammensetzungen, worin das organische Material ein Schmierstoff, insbesondere ein synthetischer Schmierstoff oder ein Schmierstoff auf Mineralölbasis ist.

In der Praxis können die erfindungsgemässen Verbindungen der Formel I auch als Gemische (wie bereits vorstehend beschrieben) oder/und zusammen mit anderen Stabilisatoren eingesetzt werden. Die Zusammensetzungen können zusätzlich noch andere Additive enthalten, die zugegeben werden, um gewisse Gebrauchseigenschaften zu verbessern, wie z.B. aminische Antioxidantien, Metallpassivatoren, Rostinhibitoren, Viskositäts-Index-Verbesserer, Stockpunkterniedriger, Dispergiermittel/Tenside und Verschleissschutzadditive.

Als weitere Additive, mit denen zusammen die erfindungsgemäss verwendeten Stabilisatoren eingesetzt werden können, sind beispielsweise zu nennen:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)- 4,6-dimethylphenol, 2,6-Di-octadecyl-4-methylphenol, 2,4,6-Tri-cyclohexylphenol, 2,6-Di-tert.butyl-4-methoxymethylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2.2'-Thio-bis-(6-tert.-butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methyl-benzyl)-4-nonylphenol], 2,2'-Methylen-bis-[6-(α,α-dimethylbenzyl)- 4-nonylphenol], 4,4'-Methylen-bis-(2,6-di-tert.butylphenol), 4,4'-Methylen-bis-(6-tert.butyl-2-methylphenol, 1,1-Bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Di-(3-tert.butyl-4-hydroxy-5-methylphenyl)-dicyclopentadien, Di-[2-(3'-tert.butyl-2'-hydroxy-5'-methyl-benzyl)-6-tert.butyl-4-methyl-phenyl]-terephthalat.

1.5 Benzylverbindungen, z.B. 1,3,5-Tri-(3,5-di-tert.butyl-4-hydroxy-benzyl)-2,4,6-trimethylbenzol, Di-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiolterephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, 3,5-Di-tert.butyl-4-hydroxybenzyl-phosphonsäure-monoethylester, Calcium-salz.

1.6. Acylaminophenole, z.B. 4-Hydroxy-laurinsäureanilid, 4-Hydroxystearinsäureanilid, 2,4-Bis-octylmercapto-6-(3,5-di-tert.butyl-4-hydroxyanilino)-s-triazin, N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.8. Ester der β-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyloxyalsäurediamid.

1.9. Amide der β-(3,5-Di-tert.butyl-4-hydroxyphenyl)-propionsäure, wie z.B. N,N'-Di-(3,5-di-tert.butyl-4-hydroxyphenylpropionyl)-hexamethylendiamin, N,N'-Di-(3,5-di-tert.butyl-4-hydroxyphenylpropionyl)-trimethylendiamin, N,N'-Di-(3,5-di-tert.butyl-4-hydroxyphenyl-propionyl)-hydrazin.

1.10 Aminische Antioxidantien, z.B. N,N'-Di-isopropyl-p-phenylendiamin, N,N'-Di-sec-butyl-p-phenylendiamin, N,N'-Bis(1,4-di-methyl-pentyl)-p-phenylendiamin, N,N'-Bis(1-ethyl-3-methyl-pentyl)-p-phenylendiamin, N,N'-Bis(1-methyl-heptyl)-p-phenylendiamin, N,N'-Diphenyl-p-phenylendiamin, N,N'-Di-(naphthyl-2)-p-phenylendiamin, N-Isopropyl-N'-phenyl-p-phenylendiamin, N-(1,3-Dimethyl-butyl)-N'-phenyl-p-phenylendiamin, N-(1-Methyl-heptyl)-N'-phenyl-p-phenylendiamin, N-Cyclohexyl-N'-phenyl-p-phenylendiamin, 4-(p-Toluol-sulfonamido)-diphenylamin, N,N'-Dimethyl-N,N'-di-sec-butyl-p-phenylendiamin, Diphenylamin, 4-Isopropoxy-diphenylamin, N-Phenyl-1-naphthylamin, N-Phenyl-2-naphthylamin, octyliertes Diphenylamin, 4-n-Butylaminophenol, 4-Butyrylamino-phenol, 4-Nonanoylamino-phenol, 4-Dodecanoylamino-phenol, 4-Octadecanoylamino-phenol, Di-(4-methoxyphenyl)-amin, 2,6-Di-tert-butyl-4-dimethylamino-methyl-phenol, 2,4'-Diamino-diphenylmethan, 4,4'-Diamino-diphenylmethan, N,N,N',N'-Tetramethyl-4,4'-diamino-diphenylmethan, 1,2-Di-[(2-methyl-phenyl)-amino]-ethan, 1,2-Di-(phenylamino-propan, (o-Tolyl)-biguanid, Di-[4-(1',3'-dimethyl-butyl)-phenyl)amin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'tert.butyl, 4'-Octoxy-, 3',5'-Di-tert.-amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenyl-salicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyldithiocarbamat, Nickelsalze von 4-Hydroxy-3,5-di-tert.butylbenzyl-phosphonsäure-monoalkylestern, wie vom Methyl- oder Ethylester, Nickelkomplexe von Ketoximen, wie von 2-Hydroxy-4-methyl-phenylundecylketonoxim, Nickelkomplexe des 1-Phenyl-4-lauroyl-5-hydroxypyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).
2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy-2,4,8,10-tetraoxa-3,9-diphosphaspiro[5.5]undecan.

5. Peroxidzerstörende Verbindungen, wie z.B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(β-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

11. Metallpassivatoren für Kupfer, z.B.:
Triazol, Benztriazol und deren Derivate, 2-Mercaptobenzthiazol, 2,5-Dimercaptothiadiazol, Salicylidenpropylendiamin, Salze von Salicylaminoguanidin.

12. Rost-Inhibitoren:

a) Organische Säuren, ihre Ester, Metallsalze und Anhydride, z.B.:
N-Oleoyl-sarcosin, Sorbitan-mono-oleat, Blei-naphthenat, Dodecenylbernsteinsäure-anhydrid, Alkenylbernsteinsäure-Halbester, 4-Nonylphenoxy-essigsäure.
b) Stickstoffhaltige Verbindungen, z.B.:
I. Primäre, sekundäre oder tertiäre aliphatische oder cycloaliphatische Amine und Amin-Salze von organischen und anorganischen Säuren, z.B. öllösliche Alkylammoniumcarboxylate.
II. Heterocyclische Verbindungen, z.B.:
Substituierte Imidazoline und Oxazoline.
c) Phosphorhaltige Verbindungen, z.B.:
Aminsalze von Phosphorsäurepartialestern.
d) Schwefelhaltige Verbindungen, z.B.:
Barium-dinonylnaphthalin-sulfonate, Calciumpetroleum-sulfonate.

13. Viskositätsindex-Verbesserer:
Polymethacrylate, Vinylpyrrolidon/Methacrylat-Copolymere, Polybutene, Olefin-Copolymere, Styrol/Acrylat-Copolymere.

14. Stockpunkterniedriger:
Polymethacrylat, alkylierte Naphthalinderivate.

15. Dispergiermittel/Tenside:
Polybutenylbernsteinsäure-imide, Polybutenylphosphonsäurederivate, basische Magnesium-, Calcium-, und Bariumsulfonate und -phenolate.

16. Verschleissschutz-Additive:
Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

Von besonderem Interesse sind Zusammensetzungen, welche mindestens eine Verbindungen der Formel I zusammen mit mindestens einem S-haltigen Additiv als Stabilisator enthalten.
Die nachfolgenden Beispiel erläutern die Erfindung weiter. In den Beispielen, der übrigen Beschreibung und den Patentansprüchen genannte Prozente und Teile sind Gewichtsprozente und Gewichtsteile.

Herstellungsbeispiele

Beispiel 1: 3,5-Bis(2'-ethylhexyloxycarbonylmethyl-thiomethyl)-2,4,6-trimethyl-phenol

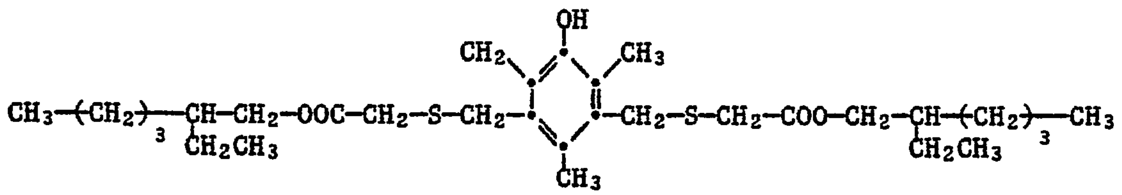

Zu einem Gemisch von 5 g 3,5-Bis(chlormethyl)mesitol, 9,5 ml 2-Mercapto-essigsäure-2-ethylhexylester und 40 ml Ethanol werden bei Raumtemperatur unter Stickstoff 9 ml Triethylamin zugetropft. Das Reaktionsgemisch wird während zwei Stunden bei Raumtemperatur gerührt, anschliessend auf 1N Salzsäure gegossen und mit Ether extrahiert.
Nach Eindampfen der Etherphase am Rotationsverdampfer erhält man 3,5-Bis(2'-ethylhexyloxycarbonylmethyl-thiomethyl)-2,4,6-trimethyl-phenyl als weisses Pulver (Smp 58-59°C) in einer Ausbeute von 93 % der Theorie.

| | | |
|---|---|---|
| Analyse: | berechnet: | S 11,27% |
| | gefunden: | S 11,27% |

Beispiel 2: 3,5-Bis(t-dodecylthiomethyl)-2,4,6-trimethyl-phenol.

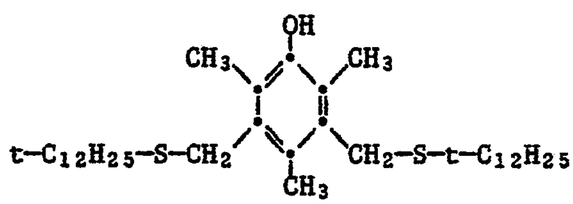

Zu einem Gemisch von 8 g 3,5-Bis(chlormethyl)mesitol, 14,6 g t-Dodecylmerkaptan und 80 ml Acetonitril werden bei Raumtemperatur unter Stickstoff 10,7 ml 1,8-Diazabicyclo(5,4,0)undec-7-en zugetropft.
Das Gemisch wird während zwei Stunden bei Raumtemperatur gerührt. Dann wird das Lösungsmittel abdestilliert und der Rückstand in Hexan/Aether 1:1 aufgenommen. Die Salze werden abfiltriert und die organische Phase zuerst mit 1N Salzsäure, dann mit Wasser gewaschen. Eindampfen am Rotationsverdampfer liefert 17 g 3,5-Bis(t-dodecylthiomethyl)-2,4,6-trimethyl-phenol als farbloses Oel, welches durch Säulenchromatographie weiter gereinigt werden kann.

Beispiel 3: 3,5-Bis(merkaptomethyl)-2,4,6-trimethyl-phenol

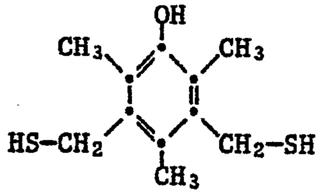

Zu einer Lösung von 42,8 g Thioharnstoff in 360 ml Diethylenglykolmonomethylaether tropft man bei 125°C und unter Stickstoff eine Lösung von 60 g 3,5-Bis(chloromethyl)-2,4,6-trimethyl-phenol in 500 ml Diethylenglykol-monomethylaether zu. Das Gemisch wird während 5 Stunden am Rückfluss erhitzt und anschliessend das Lösungsmittel am Rotationsverdampfer eingedampft.

Der feste Rückstand wird in 500 ml 2N NaOH aufgenommen und während 2 Stunden am Rückfluss erhitzt. Das erhaltene Gemisch wird dann auf 2N HCl gegossen. Das ausgefallene Produkt wird abfiltriert, mit Wasser neutral gewaschen und schliesslich bei 95°C im Vakuum getrocknet.

Man erhält 56 g (96 % der Theorie) 3,5-Bis(merkaptomethyl)-2,4,6-trimethyl-phenol als weisses Pulver, welches durch Umkristallisation noch weiter gereinigt werden kann (Smp. 159°C).

| Analyse: | berechnet: | S 28,08% |
|---|---|---|
| | gefunden: | S 28,09% |

Beispiel 4: 3,5-Bis(n-octadecyloxycarbonylethyl-thiomethyl)-2,4,6-trimethyl-phenol

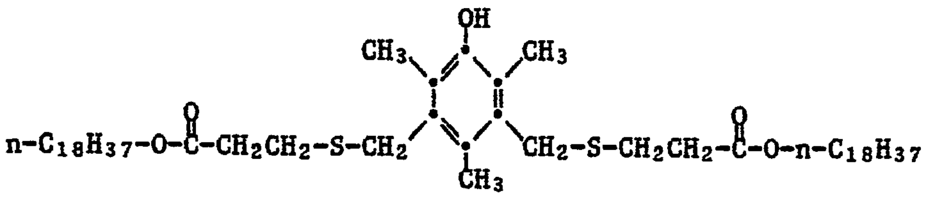

Zu einem Gemisch von 8 g 3,5-Bis(chloromethyl)-mesitol, 26 g 3-Merkaptopropionsäure-n-octadecylester, 0,43 g Dimethylaminopyridin und 120 ml eines Acetonitril/Chloroform-Gemisches (1:1) werden bei 10°C unter Stickstoff 14,5 ml Triethylamin zugetropft.

Das Gemisch wird 15 Stunden lang bei Raumtemperatur gerührt. Dann wird das Lösungsmittel abdestilliert, der Rückstand in Toluol aufgenommen und von den unlöslichen Salzen abfiltriert. Nach Eindampfen der Toluolphase erhält man 30 g 3,5-Bis(n-octadecyloxycarbonylethyl-thiomethyl)-2,4,6-trimethyl-phenol als weisses Pulver, welches durch Umkristallisation weiter gereinigt werden kann (Smp. 66-70°C).

| Analyse: | berechnet: | S 7,31% |
|---|---|---|
| | gefunden: | S 7,49% |

Beispiel 5: 3,5-Bis(2'ethylhexyloxycarbonylethyl-thiomethyl)-2,4,6-trimethyl-phenol

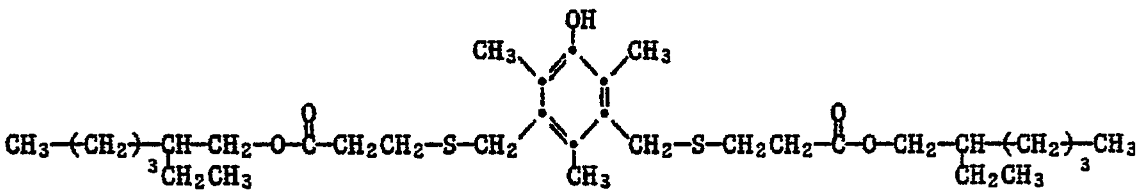

Zu einem Gemisch von 15 g 3,5-Bis(chloromethyl)mesitol, 29,3 g 3-Merkaptopropionsäure-2'ethylhexylester und 120 ml Acetonitril werden bei 0°C unter Stickstoff 26,7 ml Triethylamin zugetropft. Das Reaktionsgemisch wird während zwei Stunden bei Raumtemperatur gerührt. Die Aufarbeitung wie unter Beispiel 2, gefolgt von Säulenchromatographie liefert 32,5 g 3,5-Bis(2'-ethylhexyloxycarbonylethyl-thiomethyl)-2,4,6-trimethyl-phenol als weisses Pulver (Smp. 30-32°C).

| Analyse: | berechnet: | S 10,74% |
|---|---|---|
| | gefunden: | S 10,72% |

Beispiel 6: 3,5-Bis(t-octylthiomethyl)-2,4,6-trimethyl-phenol

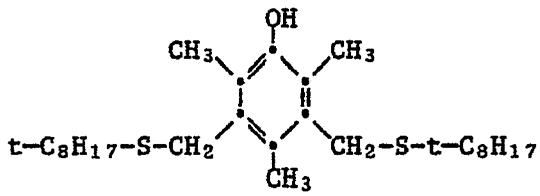

Zu einem Gemisch von 15 g 3,5-Bis(chloromethyl)mesitol, 20,7 g t-Octylmerkaptan und 120 ml Acetonitril werden bei 0°C unter Stickstoff 21,1 ml 1,8-Diazabicyclo(5,4,0)undec-7-en zugetropft. Das Gemisch wird 15 Minuten lang bei Raumtemperatur gerührt.

Nach Aufarbeitung wie unter Beispiel 2 erhält man 28 g 3,5-Bis-(t-octylthiomethyl)-2,4,6-trimethylphenol als weisses Pulver, welches durch Umkristallisation weiter gereinigt werden kann. (Smp. 98-101°C).

| Analyse: | berechnet: | S 14,16% |
|---|---|---|
| | gefunden: | S 14,24% |

Beispiel 7: 3,5-Bis(2'-ethylhexyloxycarbonylmethyl-thiomethyl)-2,4-dimethyl-6-ethylphenol

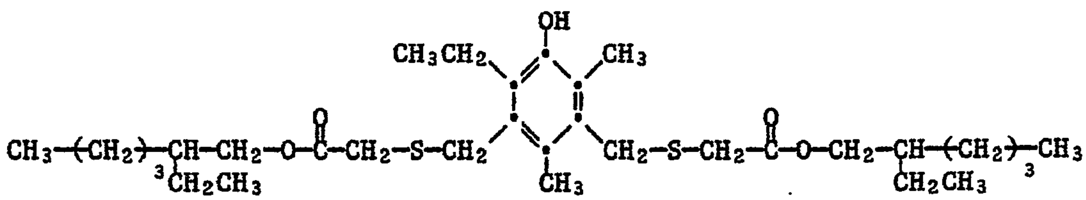

Zu einem Gemisch von 3 g 2,4-dimethyl-6-ethylphenol, 10,6 g 2-chloromethyl-merkaptoessigsäure-2'-ethylhexylester und 20 ml $CH_2Cl_2$ werden bei 0°C unter Stickstoff 4,9 ml Zinntetrachlorid zugetropft. Das Reaktionsgemisch wird während 5 Stunden bei 50°C gerührt. Nach Abkühlen wird das Reaktiosgemisch auf Wasser gegossen und mit Dichlormethan extrahiert.

Nach Eindampfen des Lösungsmittels und Reinigung des Rückstandes mit Säulenchromatographie erhält man 11 g 3,5-Bis(2'-ethylhexyloxycarbonylmethyl-thiomethyl)-2,4-dimethyl-6-ethylphenol, als hellgelbes Oel.

| Analyse: | berechnet: | S 11,0% |
|---|---|---|
| | gefunden: | S 10,89% |

Beispiel 8: 3,5-Bis[(3'-t-butyl-4'-hydroxy-5'-methyl)benzylthiomethyl]-2,4,6-trimethyl-phenol

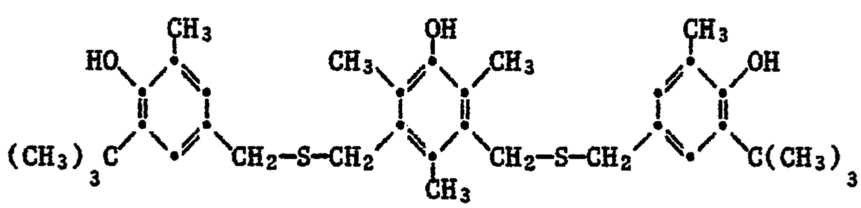

Ein Gemisch von 6 g 3,5-Bis(merkaptomethyl)-2,4,6-trimethyl-phenol, 12,8 g 2-t-Butyl-4-(N,N-dimethylaminomethyl)-6-methylphenol und 150 ml Dimethylformamid wird während 2 Stunden bei 200 Torr auf 100°C erhitzt. Nach Abkühlen wird das Reaktionsgemisch auf 1N Salzsäure gegossen und mit Aethylacetat extrahiert.

Nach Eindampfen des Lösungsmittels und Reinigung des Rückstandes mit Säulenchromatographie erhält man 12,5 g des Produktes als Pulver (Smp. 82°C).

| | | |
|---|---|---|
| Analyse: | berechnet: | S 11,04% |
| | gefunden: | S 10,77% |

Beispiel 9: 3,5-Bis[(2'6'-dimethyl-4'-t-butyl-3'-hydroxy)benzylthiomethyl]-2,4,6-trimethyl-phenol

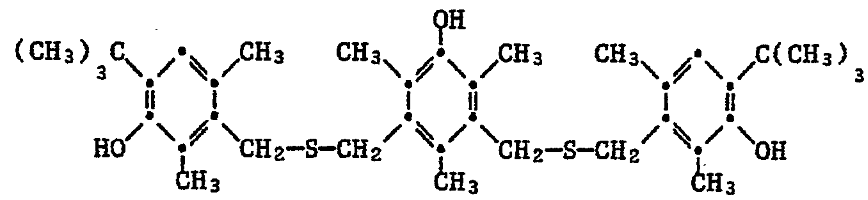

Zu einem Gemisch von 5 g 3,5-Bis(merkaptomethyl)-2,4,6-trimethylphenol, 12,9 g 6-t-Butyl-3-chlormethyl-2,4-dimethylphenol und 100 ml Dichlormethan werden bei 0°C unter Stickstoffatmosphäre 11,3 ml Triethylamin zugetropft. Das Gemisch wird anschliessend während 15 Stunden unter Rückfluss erhitzt. Die Aufarbeitung wie unter Beispiel 1, gefolgt von Säulenchromatographie liefert 9 g des Produktes als Pulver (Smp. 80°C).

| | | |
|---|---|---|
| Analyse: | berechnet: | S 10,53% |
| | gefunden: | S 10,32% |

Anwendungsbeispiele

Beispiel A: Stabilisierung von Polypropylen

100 Teile Polypropylenpulver, enthaltend 0,1 % Calciumstearat, werden mit den in der nachstehenden Tabelle aufgeführten Verbindungen gemischt und anschliessend in einem Brabander Plastographen bei 200°C 10 Minuten lang geknetet.

Die so erhaltene Masse wird in einer Presse mit einer Oberflächentemperatur von 260°C zu 1 mm dicken Platten gepresst, aus denen Streifen von 1 cm Breite und 10 cm Länge gestanzt werden. Von jeder Platte werden mehrere solcher Streifen in einem auf 135°C oder 149°C geheizten Umluftofen gehängt und in regelmässigen Zeitabständen beobachtet. Die oxidative Zersetzung dieser Streifen lässt sich an einer kreisartig beginnenden Gelbfärbung bekennen.

Ein Mass für die Stabilität der Probe ist die Zeitdauer in Tagen bis zur Zersetzung.

| Verbindung aus Beispiel | Eingesetzte Menge (%) | Anzahl Tage bis zur Zersetzung bei 135°C | bei 148°C |
|---|---|---|---|
| keine | - | 1 | <1 |
| 1 + Distearylthiodipropionat (DSTDP) | 0,1 + 0,3 | 222 | 64 |

Beispiel B: Stabilisierung von Styrol-Butadien-Copolymer

Die in der nachfolgenden Tabelle aufgeführten Verbindungen werden in wenig Methanol gelöst und in 100 Teile SBR-Latex (Styrol-Butadien-Copolymer) eingerührt. Anschliessend wird eine genau definierte Menge Latex in Petrischalen gefüllt und im Trockenschrank bei 80°C getrocknet. Man erhält transparente Filme von ca. 0,2 mm Schichtdicke.

Die Stabilität der Proben wird nach der Ofenalterung bei 135°C anhand der Gelbfärbung (Yellowness Index) in regelmässigen Zeitabständen gemessen. Die Messung von Yellowness Index erfolgt nach ASTM D 1925-70. Die Ergebnisse sind in der nachfolgenden Tabelle zusammengefasst.

| Verbindung aus Beispiel | Eingesetzte Menge (%) | Yellowness Index nach Anzahl Stunden bei 135°C | | | |
|---|---|---|---|---|---|
| | | 8 | 24 | 48 | 75 |
| keine | - | 37,6 | 64,4 | 89,6 | 107,4 |
| 8 | 0,25 | 12,9 | 16,7 | 19,8 | 23,7 |
| 9 | 0,25 | 12,7 | 19,5 | 20,9 | 28,7 |

Beispiel C: Stabilisierung von Acrylnitril-Butadien-Styrol-Copolymer (ABS)

0.25 g der zu prüfenden Verbindungen und gegebenenfalls 0,5 g eines Additivs werden in 40 ml eines Lösungsmittel-Gemisch es aus Hexan/Isopropanol gelöst. Die Lösung wird unter kräftigem Rühren zu einer Dispersion von 100 g ABS in 600 g Wasser gegeben, wobei die Lösung durch das ABS in kurzer Zeit (in einer Minute) vollständig absorbiert wird. Das ABS-Pulver wird abgenutscht und während 40 Stunden bei 40°C im Vakuum getrocknet. Dem trockenen Pulver werden 2 % Titandioxid (Pigment), sowie 1 % Ethylen-bis-stearin-säureamid (Gleitmittel) zugegeben. Die Mischung wird anschliessend während 4 Minuten auf einem Zweiwalzenstuhl bei 180°C compoundiert.

Aus dem Walzfell wird bei 175°C eine Platte von 0,8 mm Dicke gepresst, aus welcher Prüflinge der Dimension 45x17 mm ausgestanzt werden. Eine Platte ohne die erfindungsgemässe Verbindung wird auf dieselbe Weise hergestellt. Die Prüfung auf Wirksamkeit der zugesetzten Verbindung wird durch Hitzealterung in einem Umluftofen bei 180°C vorgenommen. Als Kriterium dient die Farbentwicklung nach 90 Minuten Prüfdauer. Die Farbintensität wird mit dem "Yellowness Index" nach ASTM D 1925-70 bestimmt. Höhere Zahlen bedeuten intensivere Gelbfärbung. Die Versuche zeigen, dass die Gelbfärbung durch die zugesetzten erfindungsgemässen Verbindungen wirksam unterdrückt werden.

| Verbindung aus Beispiel | Eingesetzte Menge (%) | Yellowness Index nach 90 Minuten bei 180°C |
|---|---|---|
| keine + Dilaurylthio-dipropionat (DLTDP) | 0,5 | 104 |
| keine + Distearylthio-dipropionat (DSTDP) | 0,5 | 104 |
| 8 + DLTDP | 0,25 + 0,5 | 36 |
| 9 + DLTDP | 0,25 + 0,5 | 51 |
| 2 + DSTDP | 0,25 + 0,5 | 31 |
| 6 + DSTDP | 0,25 + 0,5 | 31 |
| 1 + DSTDP | 0,25 + 0,5 | 41 |

Beispiel D: Stabilisierung von Acrylnitril-Butadien-Copolymer (NBR)

Herstellung der Vulkanisate: Mischungsherstellung bei 50°C auf Walzwerk 150 x 350; Friktion 1:1,2; Mischdauer 30 Min.;

| Rezeptur | |
|---|---|
| NBR (frei von Additiven) | 100 Teile |
| ZnO | 5 Teile |
| Stearinsäure | 0,5 Teile |
| Aktivkohle | 75 Teile |
| Dioctylphthalat | 5 Teile |
| Unlöslicher Schwefel | 1,25 Teile |
| Vulkanisationsbeschleuniger (Vulk. Thiuran MS) | 0,5 Teile |
| Stabilisator gemäss der Erfindung | 1 Teil |

Vulkanisation von 2 mm-Platten bei 166°C bis T 95.
Analogerweise wird eine Probe ohne Stabilisator hergestellt.
Alterungsprüfung: 3tägige "Alterung" in Inertgas (Argon) bei 95°C und anschliessende Ofenalterung bei 80°C.

| Verbindung aus Beispiel | Eingesetzte Menge (%) | Bruchdehnung (%) | | | |
|---|---|---|---|---|---|
| | | ohne Alterung | nach Anzahl Tagen Ofenalterung | | |
| | | | 14 | 28 | 46 |
| keine | - | 410 | 190 | 100 | 40 |
| 3 | 1,0 | 430 | 250 | 210 | 150 |

**Patentansprüche**

1. Verbindungen der Formel I

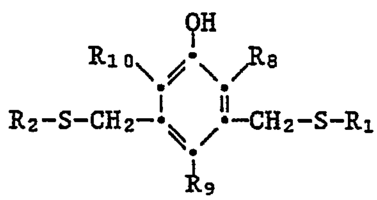

(I),

worin $R_1$ und $R_2$ unabhängig voneinander Wasserstoff, $C_1$-$C_{20}$-Alkyl, $C_3$-$C_{20}$-Alkenyl, $C_5$-$C_{12}$-Cycloalkyl, Phenyl, 1-Naphthyl, 2-Naphthyl, $C_1$-$C_4$-Alkyl-Phenyl, Phenyl-$C_1$-$C_4$-Alkyl oder eine Gruppe der Formeln Ia, Ib oder Ic

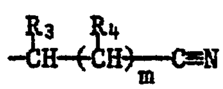 , 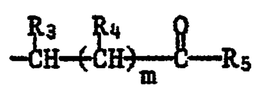 oder 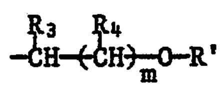

(Ia) (Ib) (Ic)

bedeuten, wobei für den Fall, dass $R_1$ und $R_2$ gleichzeitig für Alkyl stehen, mindestens einer der beiden Substituenten ein mindestens ein tertiäres C-Atom enthaltendes verzweigtes Alkyl bedeutet, oder $R_1$ und $R_2$ eine Gruppe der Formel Id

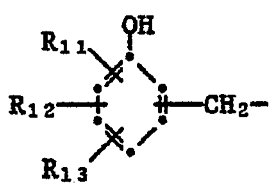

(Id)

die gleich oder verschieden sein können, bedeuten,
m Null oder 1 ist,
$R_3$ und $R_4$ unabhängig voneinander für Wasserstoff oder Methyl stehen, $R_5$ einen Rest $-OR_6$ oder $-NR_6R_7$ bedeutet,
R' für Wasserstoff oder $-CO-R_6$ steht,
$R_6$ und $R_7$ unabhängig voneinander für Wasserstoff, ein gegebenenfalls durch -O-, -S-, -NH-, $-N(CH_3)-$ oder $-N(CH_2CH_3)-$ unterbrochenes $C_1$-$C_{20}$-Alkyl, $C_2$-$C_{20}$-Alkenyl, $C_5$-$C_{12}$-Cycloalkyl, Phenyl, 1-Naphthyl, 2-Naphthyl, Phenyl-$C_1$-$C_4$-Alkyl oder $C_1$-$C_4$-Alkyl-Phenyl stehen, und
$R_8$, $R_9$ und $R_{10}$ unabhängig voneinander $C_1$-$C_{10}$-Alkyl, $C_2$-$C_{10}$-Alkenyl, $C_5$-$C_{12}$-Cycloalkyl, Phenyl, 1-Naphthyl, 2-Naphthyl, $C_1$-$C_4$-Alkyl-Phenyl oder Phenyl-$C_1$-$C_4$-Alkyl bedeuten,
$R_{11}$ $C_1$-$C_{10}$-Alkyl, $C_2$-$C_{10}$-Alkenyl, $C_5$-$C_{12}$-Cycloalkyl, $C_1$-$C_4$-Alkyl-Phenyl oder Phenyl-$C_1$-$C_4$-Alkyl ist, und
$R_{12}$ und $R_{13}$ unabhängig voneinander für Wasserstoff oder eine Bedeutung von $R_{11}$ stehen.

2. Verbindung der Formel I

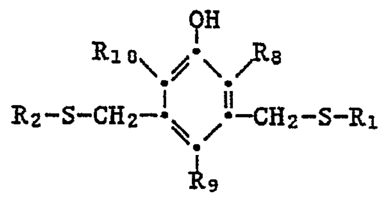

(I),

worin $R_1$ und $R_2$ unabhängig voneinander $C_1$-$C_{20}$-Alkyl, $C_3$-$C_{20}$-Alkenyl, $C_5$-$C_{12}$-Cycloalkyl, Phenyl, 1-Naphthyl, 2-Naphthyl, $C_1$-$C_4$-Alkyl-Phenyl, Phenyl-$C_1$-$C_4$-Alkyl oder eine Gruppe der Formeln Ia, Ib oder Ic

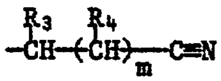 ,

(Ia)

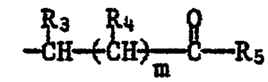 oder

(Ib)

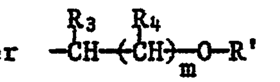

(Ic)

bedeuten, wobei für den Fall, dass $R_1$ und $R_2$ gleichzeitig für Alkyl stehen, mindestens einer der beiden Substituenten ein mindestens ein tertiäres C-Atom enthaltendes verzweigtes Alkyl bedeutet,
m Null oder 1 ist, und
R', $R_3$, $R_4$, $R_5$, $R_6$, $R_7$, $R_8$, $R_9$ und $R_{10}$ die in Anspruch 1 genannte Bedeutung haben.

3. Verbindungen der Formel I gemäss Anspruch 1, dadurch gekennzeichnet, dass $R_1$ und $R_2$ unabhängig voneinander eine Gruppe der Formel Id bedeuten.

4. Verbindung der Formel I gemäss Anspruch 3, dadurch gekennzeichnet, dass $R_{11}$ und $R_{12}$ in der Formel Id $C_1$-$C_{10}$-Alkyl bedeuten.

5. Verbindungen der Formel I gemäss Anspruch 1, dadurch gekennzeichnet, dass $R_1$ $C_3$-$C_{20}$-Alkenyl, $C_5$-$C_{12}$-Cycloalkyl, Phenyl, 1-Naphthyl, 2-Naphthyl, $C_1$-$C_4$-Alkyl-Phenyl, Phenyl-$C_1$-$C_4$-Alkyl oder eine Gruppe der Formeln Ia, Ib oder Ic bedeutet und $R_2$ $C_1$-$C_{20}$-Alkyl ist oder unabhängig von $R_1$ eine für $R_1$ genannte Bedeutung hat.

6. Verbindungen der Formel I gemäss Anspruch 1, dadurch gekennzeichnet, dass $R_1$ und $R_2$ die gleiche Bedeutung haben und für Wasserstoff, $C_6$-$C_{20}$-Alkyl mit mindestens einem tertiären C-Atom, eine Gruppe der Formel Ib oder eine Gruppe der Formel Id stehen.

7. Verbindungen der Formel I gemäss Anspruch 6, dadurch gekennzeichnet, dass in Formel Ib $R_3$ und $R_4$ Wasserstoff sind und $R_5$ für einen Rest $OR_6$ steht, worin $R_6$ $C_6$-$C_{18}$-Alkyl bedeutet und dass in Formel Id $R_{11}$ $C_1$-$C_6$-Alkyl und $R_{12}$ und $R_{13}$ unabhängig voneinander Wasserstoff oder $C_1$-$C_6$-Alkyl bedeuten.

8. Verbindungen der Formel I, gemäss Anspruch 1, dadurch gekennzeichnet, dass $R_1$ und $R_2$ die gleiche Bedeutung haben und für $C_6$-$C_{20}$-Alkyl mit mindestens einem tertiären C-Atom stehen.

9. Verbindungen der Formel I gemäss Anspruch 1, dadurch gekennzeichnet, dass $R_1$ und $R_2$ unabhängig voneinander eine Gruppe der Formel Ib bedeuten.

10. Verbindungen der Formel I gemäss Anspruch 9, dadurch gekennzeichnet, dass $R_1$ und $R_2$ unabhängig voneinander eine Gruppe

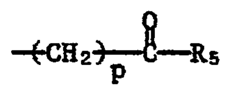 oder 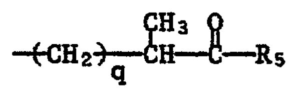

bedeuten, worin p für eine Zahl 1 oder 2 steht, q Null oder 1 bedeutet und $R_5$ die in Anspruch 1 genannte Bedeutung hat.

11. Verbindungen der Formel I gemäss Anspruch 10, dadurch gekennzeichnet, dass $R_5$ $-OR_6$ oder $-NR_6R_7$ gegebenenfalls durch -O- unterbrochenes $C_1$-$C_{18}$-Alkyl, $C_5$-$C_{12}$-Cycloalkyl, Phenyl, 1-Naphthyl, 2-Naphthyl, Phenyl-$C_1$-$C_4$-Alkyl oder $C_1$-$C_4$-Alkyl-Phenyl bedeutet, $R_7$ unabhängig von $R_6$ eine für $R_6$ angegebene Bedeutung hat, und $R_8$, $R_9$ und $R_{10}$ unabhängig voneinander $C_1$-$C_8$-Alkyl, $C_5$-$C_{12}$-Cycloalkyl, Phenyl oder Benzyl sind.

12. Verbindungen der Formel I gemäss Anspruch 1, worin $R_1$ und $R_2$ gleich sind und jeweils eine Gruppe der Formel Id bedeuten.

13. Verbindungen der Formel I gemäss Anspruch 1, dadurch gekennzeichnet, dass $R_1$ und $R_2$ $-CH_2-COO-CH_2-CH(CH_2CH_3)-(CH_2)_3-CH_3$ bedeuten, und $R_8$, $R_9$ und $R_{10}$ für Methyl stehen.

14. Verbindungen der Formel I gemäss Anspruch 1, dadurch gekennzeichnet, dass $R_1$ und $R_2$ Wasserstoff bedeuten.

15. Verfahren zur Herstellung von Verbindungen der Formel I gemäss Anspruch 1, dadurch gekennzeichnet, dass man eine Verbindung der Formel II

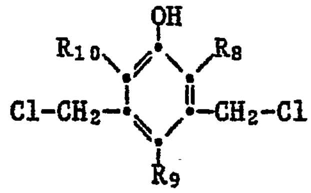

(II)

mit einem Merkaptan $R_1$-SH oder mit einem Gemisch von $R_1$-SH/$R_2$-SH in Gegenwart einer Base umsetzt, wobei $R_1$, $R_2$, $R_8$, $R_9$ und $R_{10}$ die in Anspruch 1 genannte Bedeutung haben.

16. Verwendung der Verbindungen der Formel I gemäss Anspruch 1 als Stabilisatoren gegen thermischen, oxidativen oder strahlungsinduzierten Abbau in einem organischen Material.

17. Zusammensetzungen enthaltend ein gegen thermischen, oxidativen oder strahlungsinduzierten Abbau empfindliches organisches Material und mindestens eine Verbindung der Formel I gemäss Anspruch 1.

## Claims

1. A compound of the formula I

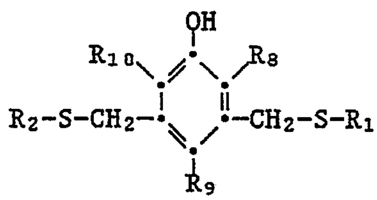

(I)

in which $R_1$ and $R_2$ independently of one another are hydrogen, $C_1$–$C_{20}$alkyl, $C_3$–$C_{20}$alkenyl, $C_5$–$C_{12}$cycloalkyl, phenyl, 1-naphthyl, 2-naphthyl, $C_1$–$C_4$alkyl-phenyl, phenyl-$C_1$–$C_4$alkyl or a group of the formula Ia, Ib or Ic

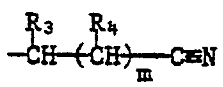 , 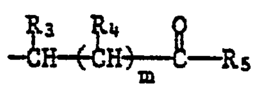 or 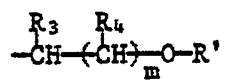 II

(Ia) (Ib) (Ic)

in which, in the case where $R_1$ and $R_2$ are simultaneously alkyl, at least one of the two substituents is a branched alkyl containing at least one tertiary C atom, or $R_1$ and $R_2$ are groups of the formula Id

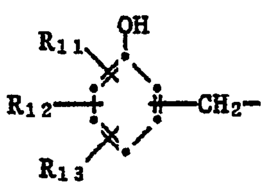

(Id)

which can be identical or different, m is zero or 1, $R_3$ and $R_4$ independently of one another are hydrogen or methyl, $R_5$ is a radical $-OR_6$ or $-NR_6R_7$, R′ is hydrogen or $-CO-R_6$, $R_6$ and $R_7$ independently of one another are hydrogen, $C_1$–$C_{20}$alkyl which may or may not be interrupted by –O–, –S–, –NH–, $-N(CH_3)-$ or $-N(CH_2CH_3)-$, $C_2$–$C_{20}$alkenyl, $C_5$–$C_{12}$cycloalkyl, phenyl, 1-naphthyl, 2-naphthyl, phenyl-$C_1$–$C_4$alkyl or $C_1$–$C_4$alkyl-phenyl, $R_8$, $R_9$ and $R_{10}$ independently of one another are $C_1$–$C_{10}$alkyl, $C_2$–$C_{10}$alkenyl, $C_5$–$C_{12}$cycloalkyl, phenyl, 1-naphthyl, 2-naphthyl, $C_1$–$C_4$alkylphenyl or phenyl-$C_1$–$C_4$alkyl, $R_{11}$ is $C_1$–$C_{10}$alkyl, $C_2$–$C_{10}$alkenyl, $C_5$–$C_{12}$cycloalkyl, $C_1$–$C_4$alkyl-phenyl or phenyl-$C_1$–$C_4$alkyl and $R_{12}$ and $R_{13}$ independently of one another are hydrogen or have one of the meanings of $R_{11}$.

2. A compound of the formula I

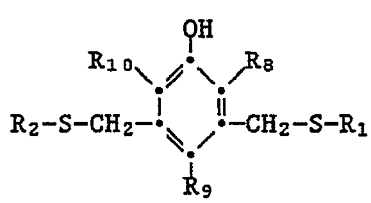

(I)

in which $R_1$ and $R_2$ independently of one another are $C_1$–$C_{20}$alkyl, $C_3$–$C_{20}$alkenyl, $C_5$–$C_{12}$cycloalkyl, phenyl, 1-naphthyl, 2-naphthyl, $C_1$–$C_4$alkylphenyl, phenyl-$C_1$–$C_4$alkyl or a group of the formula Ia, Ib or Ic

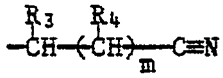 , 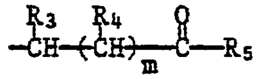 or 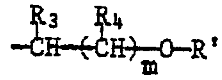

(Ia) (Ib) (Ic)

in which, in the case where $R_1$ and $R_2$ simultaneously are alkyl, at least one of the two substituents is branched alkyl containing at least one tertiary C atom, m is zero or 1 and R′, $R_3$, $R_4$, $R_5$, $R_6$, $R_7$, $R_8$, $R_9$ and $R_{10}$ are as defined in claim 1.

3. A compound of the formula I according to claim 1, wherein $R_1$ and $R_2$ independently of one another are groups of the formula Id.

4. A compound of the formula I according to claim 3, wherein $R_{11}$ and $R_{12}$ in formula Id are $C_1$–$C_{10}$alkyl.

5. A compound of the formula I according to claim 1, wherein $R_1$ is $C_3$–$C_{20}$alkenyl, $C_5$–$C_{12}$cycloalkyl, phenyl, 1-naphthyl, 2-naphthyl, $C_1$–$C_4$alkyl-phenyl, phenyl-$C_1$–$C_4$alkyl or a group of the formula Ia, Ib or Ic and $R_2$ is $C_1$–$C_{20}$alkyl or, independently of $R_1$, has one of the meanings given for $R_1$.

6. A compound of the formula I according to claim 1, wherein $R_1$ and $R_2$ have the same meaning and are hydrogen, $C_6$–$C_{20}$alkyl with at least one tertiary C atom, a group of the formula Ib or a group of the formula Id.

7. A compound of the formula I according to claim 6, wherein, in formula Ib, $R_3$ and $R_4$ are hydrogen and $R_5$ is a radical $OR_6$, in which $R_6$ is $C_6$–$C_{18}$alkyl, and in formula Id, $R_{11}$ is $C_1$–$C_6$alkyl and $R_{12}$ and $R_{13}$ independently of one another are hydrogen or $C_1$–$C_6$alkyl.

8. A compound of the formula I according to claim 1, wherein $R_1$ and $R_2$ have the same meaning and are $C_6$–$C_{20}$alkyl with at least one tertiary C atom.

9. A compound of the formula I according to claim 1, wherein $R_1$ and $R_2$ independently of one another are groups of the formula Ib.

10. A compound of the formula I according to claim 9, wherein $R_1$ and $R_2$ independently of one another are a group

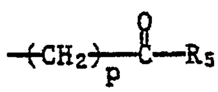 or 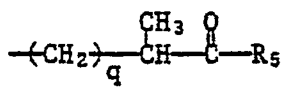

in which p is the number 1 or 2, q is zero or 1 and $R_5$ has the meaning given in claim 1.

11. A compound of the formula I according to claim 10, wherein $R_5$ is $-OR_6$ or $-NR_6R_7$, $R_6$ is $C_1$–$C_{18}$alkyl which may or may not be interrupted by –O–, $C_5$–$C_{12}$cycloalkyl, phenyl, 1-naphthyl, 2-naphthyl, phenyl-$C_1$–$C_4$alkyl or $C_1$–$C_4$alkyl-phenyl, $R_7$ independently of $R_6$ has one of the meanings given for $R_6$ and $R_8$, $R_9$ and $R_{10}$ independently of one another are $C_1$–$C_8$alkyl, $C_5$–$C_{12}$cycloalkyl, phenyl or benzyl.

12. A compound of the formula I according to claim 1, in which $R_1$ and $R_2$ are identical and each is a group of the formula Id.

13. A compound of the formula I according to claim 1, wherein $R_1$ and $R_2$ are $-CH_2-COO-CH_2-CH(CH_2CH_3)-(CH_2)_3-CH_3$ and $R_8$, $R_9$ and $R_{10}$ are methyl.

14. A compound of the formula I according to claim 1, wherein $R_1$ and $R_2$ are hydrogen.

15. A process for the preparation of a compound of the formula I according to claim 1, which comprises reacting a compound of the formula II

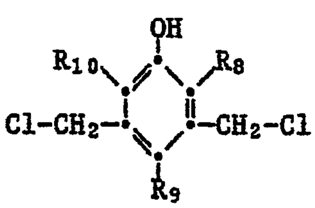

(II)

with a mercaptan $R_1$–SH or with a mixture of $R_1$–SH/$R_2$–SH in the presence of a base, $R_1$, $R_2$, $R_8$, $R_9$ and $R_{10}$ being as defined in claim 1.

16. The use of a compound of the formula I according to claim 1 as a stabilizer against degradation by heat or oxidation or induced by radiation in an organic material.

17. A composition containing an organic material which is sensitive to degradation by heat or oxidation or induced by radiation and at least one compound of the formula I according to claim 1.

## Revendications

1. Composés de formule I:

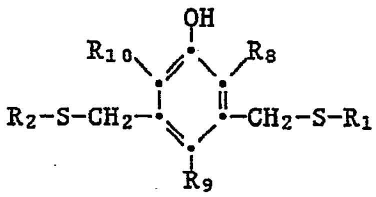

(I),

dans laquelle $R_1$ et $R_2$ représentent, indépendamment l'un de l'autre, un atome d'hydrogène, un groupe alkyle en $C_1$ à $C_{20}$, alcényle en $C_3$ à $C_{20}$, cycloalkyle en $C_5$ à $C_{12}$, phényle, naphtyle-1, naphtyle-2, alkylphényle dont le groupe alkyle comporte 1 à 4 atomes de carbone, phénylalkyle dont le groupe alkyle comporte 1 à 4 atomes de carbone, ou un groupe répondant aux formules Ia, Ib ou Ic:

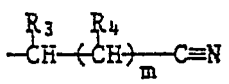

(Ia) ,

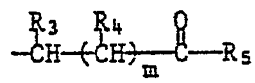

(Ib) ou

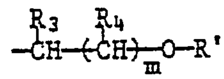

(Ic)

et, si $R_1$ et $R_2$ représentent simultanément chacun un groupe alkyle, au moins l'un des deux stabilisants représente un groupe alkyle ramifié contenant au moins un atome de carbone tertiaire,
ou bien $R_1$ et $R_2$, qui peuvent être identiques ou différents, représentent chacun un groupe de formule Id:

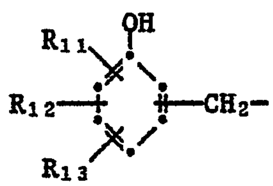

(Id)

m est nul ou vaut 1,
$R_3$ et $R_4$ représentent, indépendamment l'un de l'autre, un atome d'hydrogène ou un groupe méthyle,
$R_5$ représente un reste $-OR_6$ ou $-NR_6R_7$,
R′ représente un atome d'hydrogène ou $-CO-R_6$,
$R_6$ et $R_7$ représentent, indépendamment l'un de l'autre, un atome d'hydrogène, un groupe alkyle en $C_1$ à $C_{20}$, éventuellement interrompu par –O–, –S–, –NH–, $-N(CH_3)-$ ou $-N(CH_2CH_3)-$, un groupe alcényle en $C_2$ à $C_{20}$, cycloalkyle en $C_5$ à $C_{12}$, phényle, naphtyle-1, naphtyle-2, phénylalkyle ou alkyl-phényle dont la partie alkyle comporte à chaque fois 1 à 4 atomes de carbone,
$R_8$, $R_9$ et $R_{10}$ représentent, indépendamment l'un de l'autre, un groupe alkyle en $C_1$ à $C_{10}$, alcényle en $C_2$ à $C_{10}$, cycloalkyle en $C_5$ à $C_{12}$, phényle, naphtyle-1, naphtyle-2, alkylphényle ou phénylalkyle dont la partie alkyle comporte à la fois 1 à 4 atomes de carbone,
$R_{11}$ représente un groupe alkyle en $C_1$ à $C_{10}$, alcényle en $C_2$ à $C_{10}$, cycloalkyle en $C_5$ à $C_{12}$, alkylphényle ou phénylalkyle dont la partie alkyle comporte à chaque fois 1 à 4 atomes de carbone, et
$R_{12}$ et $R_{13}$ représentent, indépendamment l'un de l'autre, un atome d'hydrogène ou ils possèdent l'un des sens de $R_{11}$.

2. Composés de formule I:

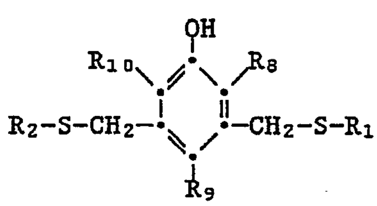

(I),

dans laquelle $R_1$ et $R_2$ représentent, indépendamment l'un de l'autre, un groupe alkyle en $C_1$ à $C_{20}$, alcényle en $C_3$ à $C_{20}$, cycloalkyle en $C_5$ à $C_{12}$, phényle, naphtyle-1, napthyle-2, alkylphényle en $C_1$ à $C_4$, phényl-alkyle (1 à 4 atomes de carbone dans la partie alkyle) ou bien un groupe correspondant aux formules Ia, Ib ou Ic:

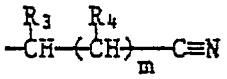 , 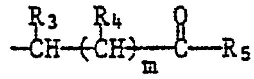 ou 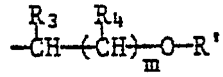

(Ia) (Ib) (Ic)

et, si $R_1$ et $R_2$ repésentent simultanément chacun un groupe alkyle, au moins l'un des deux substituants représente un groupe alkyle ramifié comportant au moins un atome de carbone tertiaire,
m est nul ou vaut 1, et R', $R_3$, $R_4$, $R_5$, $R_6$, $R_7$, $R_8$, $R_9$ et $R_{10}$ ont le sens indiqué à la revendication 1.

3. Composés de formule I selon la revendication 1, caractérisés en ce que $R_1$ et $R_2$ représentent, indépendamment l'un de l'autre, un groupe de formule Id.

4. Composés de formule I selon la revendication 3, caractérisés en ce que $R_{11}$ et $R_{12}$ représentent, dans la formule Id, un groupe alkyle en $C_1$ à $C_{10}$.

5. Composés de formule I selon la revendication 1, caractérisés en ce que $R_1$ représente un groupe alcényle en $C_3$ à $C_{20}$, cycloalkyle en $C_5$ à $C_{12}$, phényle, napthyle-1, napthyle-2, alkyle (en $C_1$ à $C_4$)-phényle, phényl-alkyle (en $C_1$ à $C_4$) ou un groupe répondant aux formules Ia, Ib ou Ic, et $R_2$ représente un groupe alkyle en $C_1$ à $C_{20}$ ou, indépendamment de $R_1$, il a l'un des sens indiqués pour $R_1$.

6. Composés de formule I selon la revendication 1, caractérisés en ce que $R_1$ et $R_2$ ont le même sens et représentent un atome d'hydrogène, un groupe alkyle en $C_6$ à $C_{20}$ comportant au moins un atome de C tertiaire, un groupe de formule Ib ou un groupe de formule Id.

7. Composés de formule I selon la revendication 6, caractérisés en ce que, dans la formule Ib, $R_3$ et $R_4$ représentent chacun un atome d'hydrogène et $R_5$ représente un reste $OR_6$, $R_6$ représentant un groupe alkyle en $C_6$ à $C_{18}$, et en ce que, dans la formule Id, $R_{11}$ représente un groupe alkyle en $C_1$ à $C_6$ et $R_{12}$ et $R_{13}$ représentent, indépendamment l'un de l'autre, un atome d'hydrogène ou un groupe alkyle en $C_1$ à $C_6$.

8. Composés de formule I selon la revendication 1, caractérisés en ce que $R_1$ et $R_2$ ont le même sens et représentent chacun un groupe alkyle en $C_6$ à $C_{20}$ comportant au moins un atome de C tertiaire.

9. Composés de formule I selon la revendication 1, caractérisés en ce que $R_1$ et $R_2$ représentent, indépendamment l'un de l'autre, un groupe de formule Ib.

10. Composés de formule I selon la revendication 9, caractérisés en ce que $R_1$ et $R_2$ représentent, indépendamment l'un de l'autre, un groupe de formule

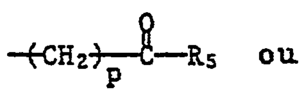 ou 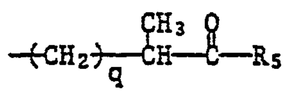

où p est un nombre valant 1 ou 2, q est nul ou vaut 1, et $R_5$ a le sens indiqué à la revendication 1.

11. Composés de formule I selon la revendication 10, caractérisés en ce que $R_5$ représente $-OR_6$ ou $-NR_6R_7$, $R_6$ représente un groupe alkyle en $C_1$ à $C_{18}$ (éventuellement interrompu par –O–), cycloalkyle en $C_5$ à $C_{12}$, phényle, naphtyle-1, napthyle-2, phénylalkyle (en $C_1$ à $C_4$) ou alkyle (en $C_1$ à $C_4$)-phényle; $R_7$ a, indépendamment de $R_6$, l'un des sens indiqués pour $R_6$, et $R_8$, $R_9$ et $R_{10}$ représentent chacun, indépendamment l'un de l'autre, un groupe alkyle en $C_1$ à $C_8$, cycloalkyle en $C_5$ à $C_{12}$, phényle ou benzyle.

12. Composés de formule I selon la revendication 1, dans lesquels $R_1$ et $R_2$ sont identiques et représentent chacun un groupe de formule Id.

13. Composés de formule I selon la revendication 1, caractérisés en ce que $R_1$ et $R_2$ représentent $-CH_2-COO-CH_2-CH(CH_2CH_3)-(CH_2)_3-CH_3$, et $R_8$, $R_9$ et $R_{10}$ représentent chacun un groupe méthyle.

14. Composés de formule I selon la revendication 1, caractérisés en ce que $R_1$ et $R_2$ représentent chacun un atome d'hydrogène.

15. Procédé pour préparer des composés de formule I selon la revendication 1, caractérisé en ce qu'on fait réagir un composé de formule II:

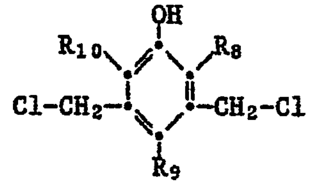

(II)

avec un mercaptan $R_1$–SH ou avec un mélange de $R_1$–SH/$R_2$–SH, en présence d'une base, les symboles $R_1$, $R_2$, $R_8$, $R_9$ et $R_{10}$ ayant le sens indiqué à la revendication 1.

16. Utilisation des composés de formule I selon la revendication 1 comme stabilisants contre une dégradation thermique, oxydante ou induite par un rayonnement dans une matière organique.

17. Compositions contenant une matière organique susceptible de subir une dégradation thermique, oxydante ou induite par un rayonnement, ainsi qu'au moins un composé de formule I selon la revendication 1.